Europäisches Patentamt

European Patent Office

Office européen des brevets

(19)

(11) Veröffentlichungsnummer: **0 100 369**
A1

# (12) EUROPÄISCHE PATENTANMELDUNG

(21) Anmeldenummer: 82106910.1

(22) Anmeldetag: 30.07.82

(51) Int. Cl.³: **A 61 M 5/31**
A 61 M 5/315, A 61 M 5/24
A 61 B 5/14

(43) Veröffentlichungstag der Anmeldung:
15.02.84 Patentblatt 84/7

(84) Benannte Vertragsstaaten:
AT BE CH DE FR GB IT LI LU NL SE

(71) Anmelder: Suwandschieff, Nikola, Dr.
13, Spitalgasse
A-6700 Bludenz(AT)

(72) Erfinder: Reitz, Günter, Dr.
Weststrasse 8
D-8671 Selbitz(DE)

(74) Vertreter: Reinhard, Kreutz & Skuhra
Leopoldstrasse 51
D-8000 München 40(DE)

(54) Vorrichtung, insbesondere zur Blutabnahme.

(57) Eine Vorrichtung, insbesondere zur Blutabnahme, oder zur Verwendung als Injektionsspritze wird durch eine Halterung (30) zur Aufnahme von Spritzenzylindern unterschiedlicher Größe gebildet und weist eine Dosiereinrichtung (38) auf, die eine Einstellung unterschiedlicher Dosiervolumina zum Aufziehen eines Reagens oder dergleichen ermöglicht. Die Halterung (30) zur Aufnahme Spritzenzylinder unterschiedlicher Größe ist nach einer ersten Ausführungsform mit mehreren konzentrisch zueinander laufenden Ringen versehen, auf welche Spritzenzylinder unterschiedlichen Durchmessers aufsteckbar sind und nach einer zweiten Ausführungsform mit meheren parallelen Schlitzen (31, 32, 33), in welche Spritzenzylinder unterschiedlichen Durchmessers und Größe mittels Flanschabschnitten einschiebbar sind. Die Einrichtung zur Dosierungseinstellung weist eine Führungskurven- und Nockeneinheit auf, die relativ zueinander verstellbar sind und abhangig von ihrer Einstellung eine Begrenzung der Kolbenstangenbewegung in Richtung auf die Öffnung des Spritzenzylinders zur Aufnahme der Nadelspitze bewirken.

Fig.7

# REINHARD , KREUTZ & SKUHRA    0100369

## PATENTANWÄLTE

Reinhard, Kreutz & Skuhra · Leopoldstraße 51 · D-8000 München 40

DR. ERNST STURM (1951-1980)
DR. HORST REINHARD
DIPL.-ING. KARL JÜRGEN KREUTZ
DIPL.-ING. UDO SKUHRA

LEOPOLDSTRASSE 51
D-8000 MÜNCHEN 40

TELEFON      : 0 89 / 33 40 78
TELEX        : 5 21 28 39 isar d
TELEGRAMM : ISARPATENT

| Unser Zeichen/our ref. | Ihr Zeichen/your ref | Datum/date |
|---|---|---|
| P1633 S/br | | 29.Juli 1982 |

Dr. Nikola Suwandschieff, Bludenz/Österreich

---

Vorrichtung, insbesondere zur Blutabnahme

---

Die Erfindung betrifft eine Vorrichtung, insbesondere zur
Blutabnahme, gemäß dem Oberbegriff des Patentanspruchs 1.
Es sind bereits Injektionsspritzen bekannt, die einen Aufbau
entsprechend dem Oberbegriff des Patentanspruchs 1 haben
und aus Metall bestehen. Weiterhin sind sogenannte Einmalspritzen aus Kunststoff bekannt, bei welchen das Medikament
in dem Spritzenzylinder mitgeliefert wird. Solche Injektionsspritzen haben aufwendige Konstruktion und werden nach einmaligem Gebrauch weggeworfen.

Die bekannten Injektionsspritzen haben generell den Nachteil,

0100369

daß ihre Halterung nur in Verbindung mit einem Spritzenzylinder einer einzigen Größe (Volumen und/oder Durchmesser) verwendbar sind und damit für unterschiedliche
Spritzenzylinder, d.h. für Spritzenzylinder verschiedenen
Inhalts jeweils verschiedene Halterungen bereitzustellen
sind.

Injektionsspritzen werden nicht nur zum Spritzen von
Medikamenten in den menschlichen Körper benutzt, sondern
auch zur Blutentnahme für diagnostische Zwecke. Hierbei
wird häufig zuerst ein Reagens in die Spritze aufgezogen,
das dann in der Spritze mit dem abgenommenen Blut vermischt wird. Wichtig ist, daß das Reagens ein genaues
Volumen einnimmt, damit das Mischungsverhältnis Reagens/Blut
stimmt. Davon hängt der diagnostische Wert der Untersuchung
ab. So werden z.B. für die Blutsenkung 0,4 ml Natrium-
Citricum-Lösung in die Spritze aufgezogen und das Blut
bis auf 2,0 ml nachgezogen. Das Mischungsverhältnis soll
1 : 5 betragen. Wird zu wenig Natrium-Citricum aufgezogen,
ist die Blutsenkung beschleunigt, wird zu viel aufgezogen,
ist die Blutsenkung verzögert. Daraus ergibt sich, daß bei
einer Ungenauigkeit des aufgezogenen Reagens das Untersuchungsergebnis verfälscht wird. Dies gilt nicht nur für
die Blutsenkung, sondern auch für alle anderen Untersuchungen, bei denen Blut mit Reagens zu mischen ist. Ein
ungenaues Volumen des aufgezogenen Reagens kann sich dadurch
ergeben, daß beim Aufziehen nicht senkrecht auf die Eichskala der Spritze geblickt wird, der Eichstrich zu dick
ist oder der Spritzenkolben verzogen ist. Auch ein relativ
großer Durchmesser des Spritzenzylinders wirkt sich auf das
meist kleine aufzuziehende Volumen an Reagens prozentual
sehr ungünstig aus und das aufgezogene Volumen stimmt bereits
dann nicht mehr, wenn der Kolben der Spritze geringfügig
von der vorgeschriebenen Eichmarke abweicht.

Der Erfindung liegt daher die Aufgabe zugrunde, eine Vorrichtung der eingangs genannten Art zu schaffen, deren Halterung zum Einsatz von Spritzenzylindern unterschiedlicher Größe verwendbar ist und bei welcher eine Begrenzung der Kolbenstangenbewegung zum Zwecke der Dosierung einer Reagenzmenge, vorzugsweise in Bezug auf die einsetzbaren Spritzenzylindergrößen, möglich ist. Diese Aufgabe wird erfindungsgemäß durch den Gegenstand des Patentanspruchs 1 gelöst.

Weitere Ausgestaltungen der Erfindung ergeben sich aus den Unteransprüchen.

Bei den heute auf dem Markt vorhandenen Einmalspritzen gibt es Spritzen mit 2,5,10 und 20 ccm, wobei sich die einzelnen Spritzenarten voneinander hinsichtlich Länge und/oder Durchmesser unterscheiden. Aus Kostengründen ist es vorteilhaft, derartige Einmalspritzen in Bezug auf die Halterung einerseits und die Spritzenzylinder andererseits aufzuteilen, so daß die Halterung mehrfach verwendbar ist, während die Spritzenzylinder nach dem Einspritzen des Medikamentes weggeworfen werden können. In diesem Fall wären für die vier vorstehend genannten Spritzentypen insgesamt vier Halterungen erforderlich. Dies führt zu relativ hohen Bereitstellungskosten.

Die Erfindung schafft eine Vorrichtung mit einer Halterung, die mit Spritzenzylindern unterschiedlicher Größe kombinierbar ist. Dadurch wird erreicht, daß für Spritzen-

- 4 -

zylinder unterschiedlicher Größe, d.h. mit unterschiedlichem Volumen und/oder Durchmesser, ein und die gleiche
Halterung verwendbar ist.

Nach einer weiteren Ausführungsform ist das Adapterelement bzw. die Halterung für Spritzenzylinder
unterschiedlicher Größe mit mehreren, vorzugsweise
drei schlitzförmigen Ausnehmungen in senkrecht zur
Kolbenstange liegenden Ebenen versehen, wobei die
schlitzförmigen Aussparungen parallel zueinanderliegend vorgesehen sind und zur Aufnahme der Flanschabschnitte Spritzenzylinder unterschiedlicher Größe
dienen.

Zur Begrenzung der Bewegung der Kolbenstange in der
Bewegungsrichtung während des Ausspritzens dient ein
mit einem Griff- oder Knopfteil in Anlage bringbares
Teil der Halterung, welches in Richtung der Achse
der Kolbenstange verstellbar und in mehreren Positionen
arretierbar ist. Damit wird erreicht, daß der Kolbenhub zumindest beim Ausspritzen abhängig von der Kolbenhub-Verstelleinrichtung begrenzt ist, und zwar derart,
daß ein vorbestimmtes Volumen, beispielsweise 0,4 ml im
Spritzenzylinder festgelegt ist, wenn die Kolbenstange bis zur Begrenzung bzw. bis zum Anschlag in Ausspritzrichtung betätigt ist. Wenn die Halterung für drei
Spritzenzylinder unterschiedlicher Größe ausgelegt ist,
wird die Einrichtung zur Begrenzung der Kolbenstangenbewegung ebenfalls auf vorzugsweise drei einstellbare
Begrenzungsstellungen konzipiert, so daß abhängig von
der Größe des Spritzenzylinders jeweils eine zugehörige
Einstellmöglichkeit erreichbar ist und somit bei jeder
Spritzenzylindergröße ein Reagenzvolumen von beispielsweise 0,4 ml vorgebbar ist.

Der universelle Einsatz der erfindungsgemäßen Halterung,z.B.
für Injektionsspritzen,führt zu einer beträchtlichen
Kostenreduzierung, da die erfindungsgemäßen Halterungen
für verschiedene Spritzengrößen einsetzbar sind und nicht
weggeworfen werden müssen. Wesentlich ist auch, daß bei
der erfindungsgemäßen Halterung die Kolbenstange in einer
Schutzeinrichtung geführt ist,wodurch eine Kontamination
der Kolbenstange und eine damit verbundene Übertragung
von Keimen von der Kolbenstange zur Innenwand des Spritzenzylinders vermieden wird.Der Einsatz der erfindungsgemäßen
Halterung für Spritzenzylinder unterschiedlicher Größe
führt zu einer Materialeinsparung von etwa 50%, die aus
der nahezu unbegrenzten, wiederholbaren Einsatzmöglichkeit der Halterung resultiert.Gegenüber Einmalspritzen
wird jedoch noch der Vorteil erreicht, daß sich hinsichtlich der Verpackung eine Materialersparnis von ca. 30%
ergibt, da nur die Spritzenzylinder, also nicht die gesamte
Spritzeneinheit, mit der Verpackung geliefert werden
müssen. Die sich ergebende Materialersparnis von etwa
50% durch eine einzige Halterung für Spritzenzylinder
unterschiedlicher Größe ist insbesondere im Hinblick
auf eine etwaige Materialknappheit von Kunststoff von
großer Bedeutung. Ein weiterer Vorteil ist, daß der praktizierende Arzt bei Einsatz der erfindungsgemäßen
Injektionsspritze eine einzige Halterung mit sich führen
muß.

Die Vorrichtung eignet sich insbesondere vorteilhafterweise
zur Blutabnahme,indem in bekannter Weise mittels einer
Nadel Blut aufgezogen wird, indem der Spritzenkolben
mittels der Kolbenstange in einer Richtung entgegengesetzt
zur Spritzennadel bewegt wird. Vor dem Aufziehen von Blut
wird im allgemeinen in eine derartige Vorrichtung ein
Reagens vorbestimmter Dosierung aufgezogen, wobei die
Vorrichtung eine exakte Dosierung des Reagenzvolumens bei
jeder, abhängig von der Halterungsart, möglichen einsatzfähigen Spritzenzylindergröße sicherstellt; so ist beispielsweise für jede der vorzugsweise drei möglichen
Spritzenzylindergrößen die gleiche Reagenzmenge dosierbar oder es sind auch mehrere verschiedene Mischungsverhältnisse einstellbar. Beim Ausspritzen des Spritzenzylinders der Vorrichtung bleibt vorteilhafterweise kein Restvolumen im Spritzenzylinder, wenn vor dem Ausspritzen
des Gemisches aus Blut und Reagens die Dosiereinheit in
ihre Nullstellung zurückverlagert wird.

Besonders vorteilhaft ist, daß nach dem Aufziehen des
Spritzenzylinders mit Blut der Spritzenzylinder selbst
als Transportmittel und gegebenenfalls als in eine Zentrifuge einzusetzender Behälter verwendbar ist.

Weiterhin ist vorgesehen, daß die Kolbenstange an ihrem
den Kolben aufnehmenden Ende nach Art einer Kupplung ausgebildet ist, so daß nach Einsetzen des Spritzenzylinders
in die Halterung der Kolben des Spritzenzylinders mit der
in der Halterung angeordneten Kolbenstange in Eingriff verbracht bzw. die Kolbenstange außer Eingriff zum Kolben zum
Zwecke der Entnahme des Spritzenzylinders aus der Halterung
gebracht werden kann.

Obgleich die Vorrichtung bevorzugt Anwendung zur Blutabnahme bzw. als Blutabnahmegerät findet, ist sie auch als
Injektionsspritze einsatzfähig, weshalb im folgenden die
erfindungsgemäße Vorrichtung teilweise auch als Injektionsspritze bezeichnet ist.

Im folgenden werden bevorzugte Ausführungsformen der erfindungsgemäßen Vorrichtung anhand der Zeichnung zur
Erläuterung weiterer Merkmale beschrieben.

Es zeigen:

Fig. 1a bis 1c verschiedene Ansichten einer ersten Ausführungsform einer Adaptereinheit zur Halterung
        von unterschiedlichen Spritzenzylindern,

Fig. 2  eine Aufsicht auf eine Vorrichtung entsprechend
        Fig. 1a,

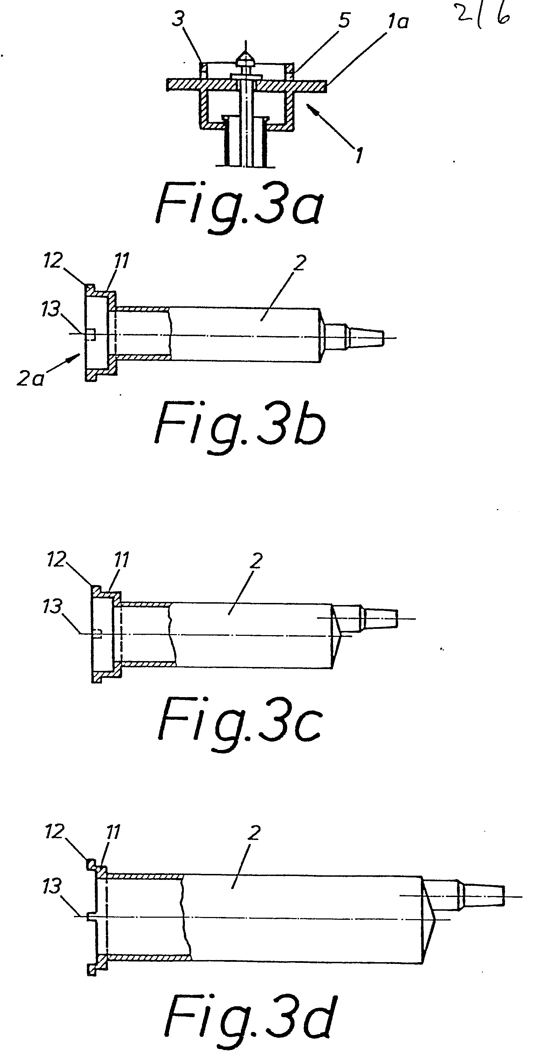

Fig. 3a eine perspektivische Teilansicht einer gegenüber
        Fig. 1a abgewandelten Ausführungsform der Halterung,

Fig. 3b bis 3d Spritzenzylinder unterschiedlicher Größe
        die in die Halterung nach Fig. 3a einsetzbar sind,

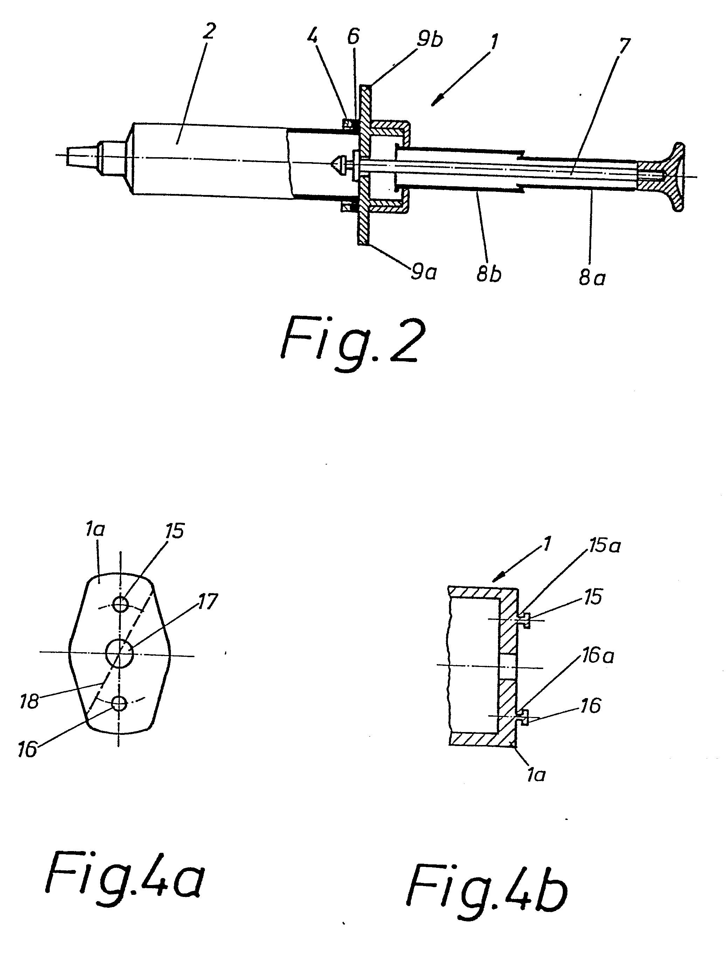

Fig. 4a und 4b eine Aufsicht und eine Teilschnittansicht
        einer abgewandelten Halterung,

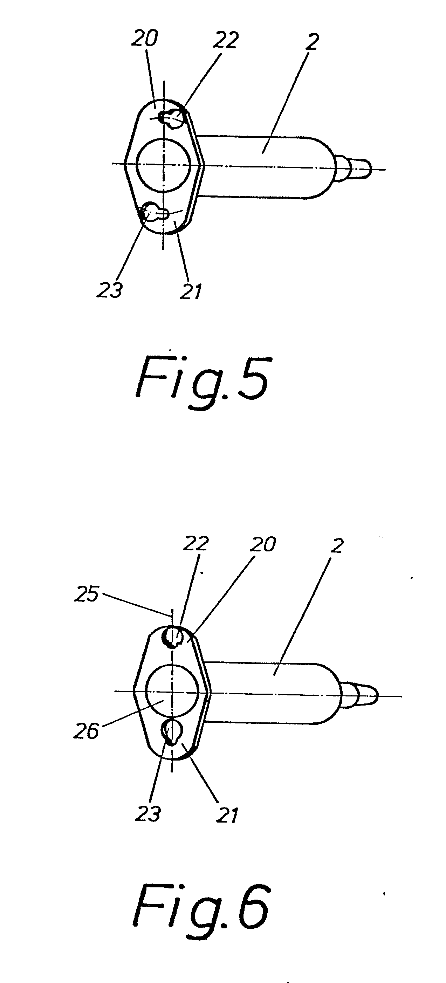

Fig. 5  eine Perspektivansicht eines Spritzenzylinders zum
        Einsatz in der Halterung nach Fig. 4,

Fig. 6    eine weitere Abwandlung eines Spritzenzylinders
          zur Kombination mit einer Halterung nach Fig. 4,

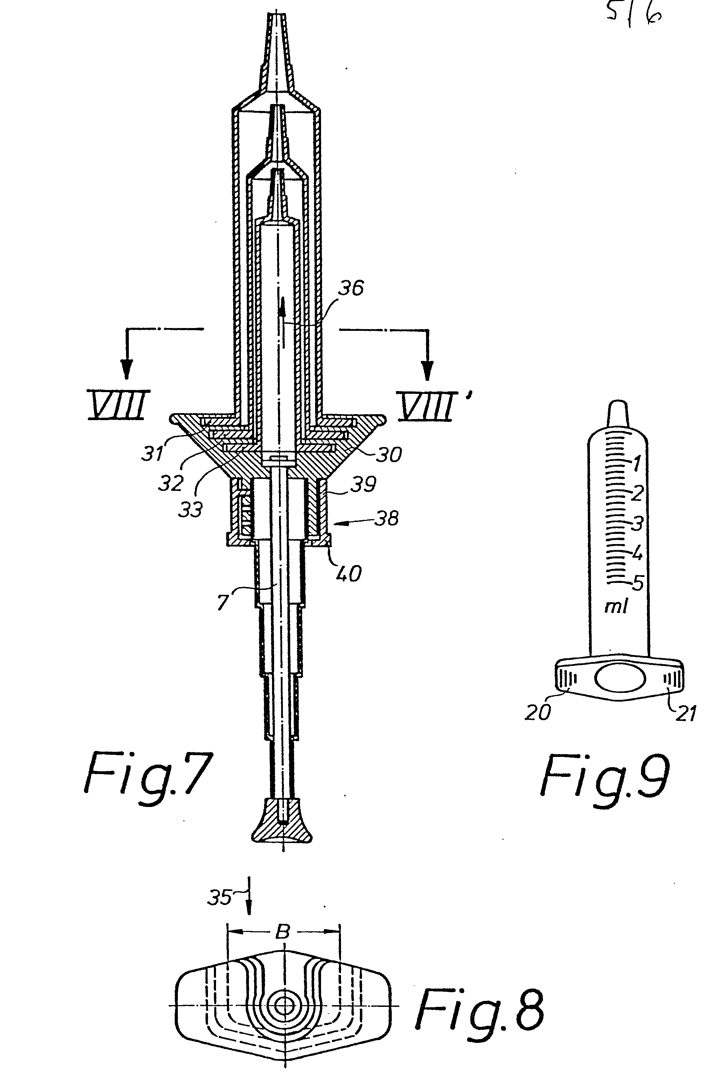

Fig. 7    eine schematische Schnittdarstellung einer weiteren
          Abwandlung einer Halterung für drei Spritzenzylinder unterschiedlicher Größe, wobei zur Veran-
          schaulichung die drei Spritzenzylinder unter-
          schiedlicher Größe eingesetzt dargestellt sind,

Fig. 8    eine Ansicht entsprechend der Linie VIII-VIII' in
          Fig. 7 zur Verdeutlichung der Halterung mit den
          Schlitzen zur Aufnahme der Flansche der Spritzenzylinder,

Fig. 9    eine Perspektivansicht eines herkömmlichen Spritzenzylinders, der in die Halterung nach Fig. 7 und 8
          einsetzbar ist,

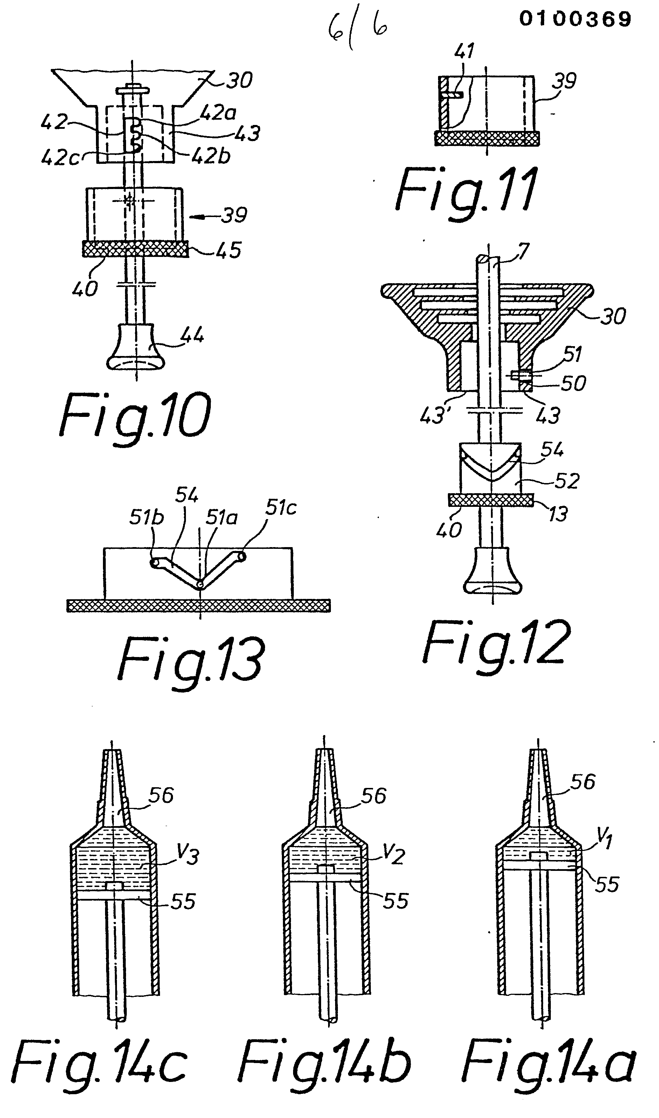

Fig.10    eine auseinandergezogene Darstellung einer Ausführungsform einer Dosiereinrichtung, wie sie in
          Verbindung mit Fig. 7 gezeigt ist,

Fig.11    eine Teilschnittansicht eines Teils der Dosiereinrichtung mit Arretierstift,

Fig.12    eine weitere Ausführungsform einer Dosiereinrichtung,

Fig.13    eine Abwicklung eines Teils der Dosiereinrichtung
          nach Fig. 12, und

Fig.14    Darstellungen zur Erläuterung von drei unterschiedlichen Reagenzvolumina.

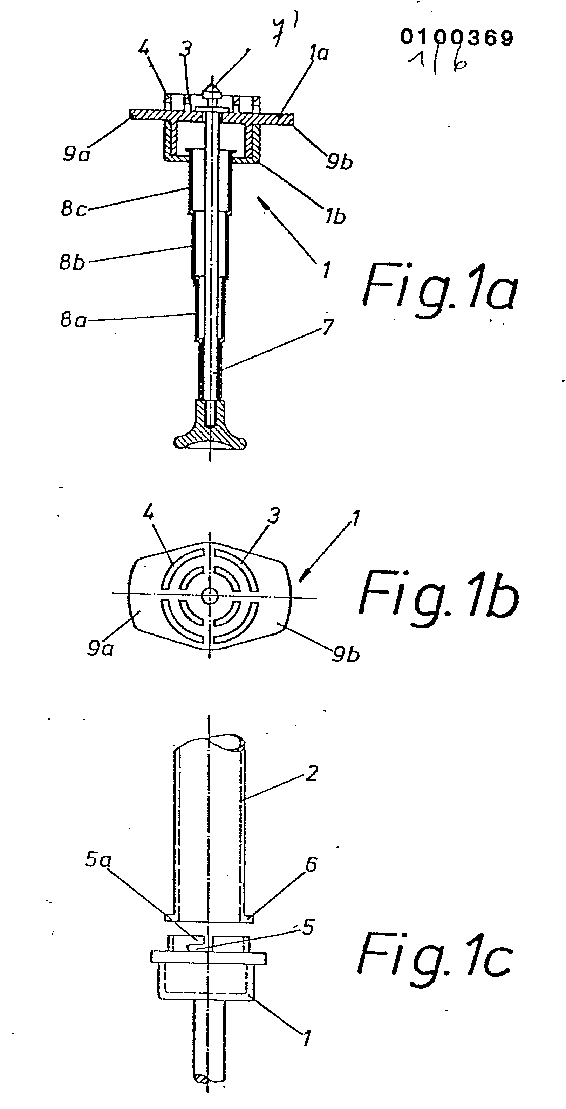

Unter Bezugnahme auf die Figuren 1a bis 1c wird im folgenden eine erste Ausführungsform einer Vorrichtung beschrieben. Die Vorrichtung, z.B. eine Injektionsspritze, besteht aus einer Halterung 1 und einem Spritzenzylinder 2 (Fig. 1c), wobei der Spritzenzylinder 2 an der Halterung 1 befestigbar ist. Gemäß dieser Ausführungsform weist die Halterung 1 an ihrer dem Spritzenzylinder 2 zugekehrten Fläche mehrere konzentrische Ringe auf, von denen in Fig.1a und 1b nur zwei Ringe 3,4 angedeutet sind. Die Ringe 3,4 haben gleiche Höhe (Fig. 1a) und sind zumindest an zwei einander diametral gegenüberliegenden Seiten mit Rastöffnungen oder Rastschlitzen 5 versehen, in welche entsprechend ausgebildete Rastnocken 6 des Spritzenzylinders 2 einrastbar sind. Die Verbindung zwischen Spritzenzylinder 2 und Halterung 1 mittels der Rastnocken 6 und der Rastöffnungen 5 erfolgt ersichtlicherweise nach Art eines Bajonettverschlusses. Die Rastnocken 6 sind integrale Elemente des Spritzenzylinders 2 und stehen unter einem Winkel von etwa 90° zur Zylinderachse vom Spritzenzylinder 2 ab.

Wie den Figuren 1a und 1b entnehmbar ist, besteht die Halterung 1 aus einem plattenförmigen Teil 1a, auf welches gemäß einer Ausführungsform ein zylindrisches Teil 1b aufgesetzt ist, welches eine nicht weiter bezeichnete Öffnung zur Durchführung einer Kolbenstange 7 aufweist. Eine entsprechende Öffnung ist in dem Plattenteil 1a ausgebildet. Die Kolbenstange 7 wird vorteilhafterweise von teleskopartig ineinander verschiebbaren Hülsenteilen 8a, 8b und 8c umgeben, die entsprechend Fig. 1a derart angeordnet sind, daß die Kolbenstange in Fig. 1a in Richtung auf das Plattenteil 1a bei Ineinanderschieben der Hülsenteile 8a bis 8c verlagerbar ist. Die Hülsenteile 8a bis 8c vermeiden eine Kontamination der Kolbenstange 7 und damit eine
Übertragung von Keimen von der Kolbenstange 7 in das
Innere des Spritzenzylinders 2.

Die Ringe 3,4 sind auf der dem zylindrischen Teil 1b
gegenüberliegenden Fläche des Plattenteiles 1a ausgebildet, wobei die Teile 1a,1b integrale Elemente der
Halterung sind.

Die in Fig. 1a gezeigte Halterung mit zwei Ringen 3,4
ermöglicht den Einsatz von Spritzenzylindern 2 mit
unterschiedlichen Größen bzw. unterschiedlichen Durchmessern, wobei der Außendurchmesser eines Spritzenzylinders 2 dem Innendurchmesser des Ringes 3 entspricht,
während ein am Ring 4 befestigbarer Spritzenzylinder
einen Außendurchmesser hat, der im Innendurchmesser des
Ringes 4 entspricht. In Fig. 1c ist der Einsatz eines
Spritzenzylinders 2 angedeutet, welcher einen Außendurchmesser gleich dem Innendurchmesser des Ringes 4 hat.
Zum Einsetzen des Spritzenzylinders 2 ist es erforderlich, den Spritzenzylinder 2 derart in den Ring 4 hineinzuschieben, daß seine Rastnocken 8 in den vertikalen
Öffnungsbereich des Schlitzes 5 hineingeschoben werden,
wonach der Spritzenzylinder 2 in Fig.1c geringfügig nach
links gedreht wird, so daß seine Rastnocken 6 am linken
Ende des Schlitzes 5 zur Anlage kommen. Ein Rastvorsprung 5a verhindert in diesem Zustand eine Entfernung
des Spritzenzylinders 2 aus der Halterung 1.

Nach einer weiteren Ausgestaltung der Erfindung können
die Ringe 3,4 mit jeweils vier Schlitzen 5 versehen sein,
was eine sichere Halterung des Spritzenzylinders 2 in
der Halterung 1 gewährleistet.

Fig. 1b zeigt ferner, daß das Plattenteil 1a eine an
die Form einer Ellipse angenäherte Gestalt besitzt und
auf diese Weise seitlich verlängerte Griffabschnitte
9a,9b trägt, die bei aufgesetztem Spritzenzylinder 2
den beim Spritzen ausgeübten Gegendruck von Zeige-
und Mittelfinger aufzunehmen gestatten. Das Plattenteil 1a kann jedoch auch jede andere, erforderliche
Gestalt haben.

Fig. 2 zeigt eine Aufsicht auf eine Vorrichtung
entsprechend der Ausführungsform nach Fig. 1a bis 1c,
wobei nur zwei Hülsenteile 8a und 8b vorgesehen sind.
Die Halterung 1 wird vorzugsweise aus Metall hergestellt,
läßt sich jedoch ohne weiteres auch aus Kunststoff fertigen.
Die Hülsenteile 8a bis 8c können entsprechend aus Metall
oder Kunststoff bestehen.

Eine weitere Ausführungsform der erfindungsgemäßen
Vorrichtung als Abwandlung zu der Ausführungsform nach
Fig. 1a bis 1c wird nachfolgend unter Bezugnahme auf
die Fig. 3a bis 3d beschrieben. Die Halterung ist in Fig.
3a in Teilschnittansicht dargestellt und mit 1 bezeichnet.
Die Halterung 1 weist im Gegensatz zu Fig. 1a nur einen
einzigen Ring 3 mit entsprechenden Rastschlitzen 5 auf,
wobei der Ring 3 an dem Plattenteil 1a in gleicher Weise,
wie unter Bezugnahme auf Fig. 1b beschrieben, angeformt
ist. Die Kombination der Halterung 1 mit verschiedenen
Spritzenzylindern 2 wird dadurch ermöglicht, daß jeder
Spritzenzylinder 2 unabhängig von seinem Volumen bzw.
seiner Größe an seinem offenen Ende 2a einen Flanschabschnitt 11 aufweist, an dem seitlich etwa senkrecht
zur Achse des Spritzenzylinders abstehende Rastnocken 12
ausgebildet sind. Bei dieser Ausführungsform ist wesentlich, daß die Flanschabschnitte 11 der Spritzenzylinder 2 unterschiedlicher Größe zumindest im Bereich ihrer Rastnocken 12 jeweils gleichen Außendurchmesser haben,
wodurch gewährleistet ist, daß die Rastnocken 12 unabhängig von der Größe des jeweiligen Spritzenzylinders
in gleicher Distanz zur mit dem Bezugszeichen 13 angedeuteten Achse des Spritzenzylinders 2 vorgesehen sind.
Vorzugsweise ist jedoch der Flanschabschnitt 11 von
zylindrischer Form, wobei sein Außendurchmesser analog
zur Ausführungsform nach Fig. 1a bis 1c dem Innendurchmesser des Ringes 3 entspricht. Das Einsetzen des Spritzenzylinders 2 erfolgt in gleicher Weise, wie unter Bezugnahme auf Fig.1a bis 1c beschrieben ist, indem der Spritzenzylinder 12 mittels seines Flanschabschnittes 11 in den
Ring 3 eingesetzt wird, wobei die Rastnocken 12 in den
in Fig. 1c dargestellten vertikalen Öffnungsbereich eingeführt werden, bis der Spritzenzylinder 2 etwa in Anlage
an die zu dem Ring 3 gewandte Fläche des Plattenteils 1a
gelangt; dann wird der Spritzenzylinder 2 derart gedreht,
daß seine Rastnocken 12 unter den Rastabschnitt 5a verbracht
werden.

Fig. 3a bis 3d zeigt Spritzenzylinder 2 verschiedener Größe.
Der Außendurchmesser des Flanschabschnittes 11 ist jeweils
gleich groß und trägt die Rastnocken 12.

Bei der Ausführungsform nach Fig. 1a bis 1c bilden die
Ringe 3,4 einen "Adapter" für Spritzenzylinder unterschiedlicher Größe, während bei der Ausführungsform nach Fig.3a
bis 3d dieser Adapter durch den Flanschabschnitt 11 am
Spritzenzylinder 2 direkt ausgebildet ist. Die Spritzenzylinder 2 bestehen in bekannter Weise aus Kunststoff.

In Fig. 4a und 4b ist eine weitere Ausführungsform einer
Halterung für die Vorrichtung dargestellt. Die Kolbenstange und die gegebenenfalls vorgesehenen Hülsenteile
sind in Fig. 4a und 4b nicht gezeigt. Bei dieser Ausführungsform weist die Halterung 1 an ihrem Plattenteil 1a etwa

- 13 -

parallel zur Achse der Vorrichtung verlaufende Rastnocken 15,16 auf, die bei dem in Fig.4a gezeigten, stehenden Plattenteil 1a vertikal übereinanderliegen. Die Rastnocken 12 können auch in anderer Anordnung, vorzugsweise
auf gleichem Radius gegenüber einer mit 17 bezeichneten
Öffnung zur Durchführung der Kolbenstange vorgesehen sein.
Damit können die Rastnocken 15,16 beispielsweise auf einer
mit 18 angedeuteten Linie liegen, wobei ersichtlicherweise auch die Zahl der Rastnocken 15,16 erhöht sein kann.
Bei dieser Ausführungsform werden vorzugsweise solche
Spritzenzylinder 2 verwendet, wie sie in den Fig. 5 und 6
angedeutet sind. Die Spritzenzylinder 2 weisen nach Fig.5
und 6 den unter Bezugnahme auf Fig. 1b beschriebenen Griffabschnitten 9a und 9b entsprechende Griffabschnitte 20,21
auf, in denen Rastöffnungen 22 und 23 ausgebildet sind.
Bei dem Spritzenzylinder nach Fig. 5 verlaufen die Rastöffnungen 22,23 im wesentlichen auf einem Kreisbogen um
die Mitte der Zylinderöffnung des Spritzenzylinders 2 und
werden durch einen etwa kreisförmigen ersten Abschnitt
gebildet, welcher in seiner Größe dem Kopf der Rastnocken
15,16 entspricht, d.h. das Einsetzen der Rastnocken 15,16
erlaubt, und einem zweiten Abschnitt, der hinsichtlich
seiner Größe reduziert ist und etwa der Größe des Halsabschnittes 15a bzw. 16a der Rastnocken entspricht.Damit läßt
sich der Spritzenzylinder 2 dadurch an der Halterung 1
befestigen, daß der Spritzenzylinder mit den Rastöffnungen
22,23 auf die Rastnocken 15,16 aufgesetzt und nach Durchtreten der Kopfabschnitte der Rastnocken 15,16 durch die
ersten Öffnungsabschnitte so verdreht wird, daß die Kopfabschnitte jeweils hinter dem zweiten Abschnitt der Rastöffnungen zu liegen kommen.

Die Ausführungsform nach Fig. 6 unterscheidet sich gegenüber Fig. 5 lediglich dadurch, daß die Rastöffnungen 22,
23 entlang einer Geraden 25 verlaufen und bei der Arretierung
des Spritzenzylinders 2 im Gegensatz zur Ausführungsform
nach Fig. 5 keine Drehbewegung, sondern eine Verschiebung entlang der Geraden 25 auszuführen ist, die nach Fig.6
die Achse des Spritzenzylinders schneidet.

Beim Einsatz von Spritzenzylindern 2 mit Griffabschnitten bzw. Flanschansätzen 20,21 ist vorgesehen, daß die
Form und Größe der Griffabschnitte 20,21 bei allen Spritzenzylindern 2 unterschiedlichen Typs bzw. unterschiedlichen
Volumens gleich groß ist.Bei einem Spritzenzylinder 2
kleineren Durchmessers ist damit die mit dem Bezugszeichen 26 angedeutete zylindrische Öffnung von kleinerem
Durchmesser als in Fig.6 dargestellt. Die Kombination der
Halterung 1 mit Spritzenzylindern 2 unterschiedlicher Größe
bzw. unterschiedlichen Durchmessers wird dadurch erreicht,
daß die Rastöffnungen 22,23 jeweils in gleicher Distanz
zum Mittelpunkt der Öffnung 26 liegen, so daß der durch
die Griffabschnitte 20,21 gebildete Flanschabschnitt die
Funktion eines Adapters hat und an diesen Flanschabschnitt
die Spritzenzylinder 2 mit unterschiedlichem Durchmesser
angeformt sind.

Fig. 7 zeigt in schematischer Schnittansicht eine weitere
Ausführungsform einer Halterung, die mit 30 bezeichnet
ist und wobei die dargestellte Ausführungsform zur Aufnahme
von drei Spritzenzylinder unterschiedlicher Größe dient.
Ersichtlicherweise wird jeweils nur ein Spritzenzylinder
eingesetzt. Die drei in Fig. 7 gezeigten Spritzenzylinder
dienen nur der Erläuterung. Die Halterung 30, die in Fig.8
in Aufsicht entlang der Linie VIII-VIII' gezeigt ist,
weist drei Schlitze 31,32,33 auf, wobei die Schlitze bzw.
die Ebenen der Schlitze jeweils senkrecht zur Achse der
Kolbenstange 7 verlaufen und wobei die Schlitze 31,32,33
gemäß Fig. 8 unterschiedliche Breite haben, wie dies
in Fig. 8 durch den Buchstaben B dargestellt ist. Der
Schlitz 33, der der Kolbenstange 7 in Fig.7 zugewandt ist,
hat dabei die kleinste Breite B und der Schlitz 31 die
größte Breite B. Diese Schlitze dienen zur Aufnahme der
Flanschabschnitte 20,21 eines herkömmlichen und in Fig.9 dargestellten Spritzenzylinders, wobei der Spritzenzylinder mit diesen Flanschabschnitten in Fig. 8 in
Richtung eines Pfeiles 35 in den jeweils zugehörigen
Schlitz 31,32 oder 33 eingeschoben wird. Die Schlitze 31
bis 33 liegen somit parallel hintereinander in der
Halterung 30. Ersichtlicherweise können mehr oder weniger
Schlitze als dargestellt vorgesehen werden abhängig von
den Anforderungen an die dargestellte Halterung 30.

Im folgenden wird eine Dosiereinrichtung, die eine Begrenzung der Bewegung der Kolbenstange während der
Ausspritzbewegung, d.h. während der Verschiebung der
Kolbenstange 7 in Richtung des Pfeiles 36 ermöglicht, erläutert.
Die Dosiereinrichtung 38 wird nachfolgend unter Bezugnahme auf die Halterung 30 erläutert, wobei die Dosiereinrichtung 38 sich bei jeder der vorstehend unter Bezugnahme auf die Fig. 1 bis 7 beschriebenen Halterungen
anwenden läßt. Die Dosiereinrichtung besteht gemäß Fig.7
und 10 aus einem zylindrischen Element 39 und einem den
Boden des zylindrischen Elementes 39 bildenden Teiles 40,
wobei im Boden 40 eine mittige Bohrung zur Durchführung
der Kolbenstange 7 und der gegebenenfalls vorgesehenen
Schutz- oder Hülsenteile 8a bis 8c dient. In dem zylindrischen Teil 39 ist ein von der Wandung des zylindrischen
Teiles nach innen weisender Stift 41 vorgesehen, der entlang von Rastnuten 42 verschiebbar ist, welche in einem
auf die Halterung 30 aufgesetzten ringförmigen Teil 43
ausgebildet sind. Der Ring 43 kann ein integrales Teil
der Halterung 30 darstellen. Wie aus Fig. 10 hervorgeht,
ist damit das zylindrische Teil 39 höhenmäßig gegenüber
dem Ring 43 verstellbar und jeweils arretierbar, indem
der Stift 41 in eine der Arretierungsnuten 42a,42b oder
42c verlagert wird. Der Zusammenbau der Dosiereinrichtung
30 erfolgt zweckmäßigerweise derart, daß der Stift 41
erst dann klemmend in das zylindrische Teil 39 eingesetzt
wird, wenn das Teil 39 mit der Öffnung für den Stift 41 über dem Ring 43 sitzt bzw. die Öffnung für den Stift 41 in Flucht zur Arretierungsnut 42 liegt. Durch Abschleifen der Außenwand des zylindrischen Teils 39 läßt sich der Stift 41 in der in Fig. 11 gezeigten Weise in der Wandung des zylindrischen Teiles 39 fest anordnen. Es ist weiter zu beachten, daß ein am Ende der Kolbenstange 7 angebrachter Knopf oder Griff 44 auf dem zylindrischen Teil 39 bzw. dessen Wand 40 zur Anlage kommt und nicht durch die in der Wand 40 ausgebildete mittige Öffnung hindurchgehen darf. Abhängig von der vorgenommenen Einstellung des zylindrischen Elementes 39 infolge des Einrastens des Stiftes 41 in einer der Nuten 42a, 42b oder 42c wird damit die Endlage der Kolbenstange 7 mit dem Knopf 44 in Richtung des Pfeiles 36 (Fig. 7) bestimmt, d.h. der Abstand zwischen dem Knopf 44 und der Halterung 30 ist am größten, wenn der Stift 41 in der Nut 42c einrastet und am kleinsten, wenn der Stift 41 in die Nut 42a eingeschoben ist. Entsprechend ergibt sich ein unterschiedlich großes Dosiervolumen in dem zugehörigen Spritzenzylinder, wie dies noch unter Bezugnahme auf Fig. 14 beschrieben ist. Zur leichten Handhabung des zylindrischen Teiles 39 ist dieses mit einer Rändelung 45 versehen, wodurch das teilweise Drehen gegenüber der Achse der Kolbenstange 7 und das Verschieben des zylindrischen Teiles 39 in Richtung der Achse der Kolbenstange 7 und ein besseres Erfassen des zylindrischen Teiles 39 sichergestellt sind.

Fig. 12 und 13 zeigen eine weitere Ausführungsform einer Dosiereinrichtung, welche der Halterung 30 zugeordnet ist. Die Halterung weist ein zylinderförmiges Teil 43 mit einer Gewindeöffnung 50 zur Aufnahme eines Schraubstiftes 51 auf. In das Teil 43, das integral zur Halterung 30 ausgebildet sein kann, wird ein hülsenförmiges Teil 52 mit einer Rändelung 53 eingesetzt. Das Teil 52 weist eine über einen Teilbogen des hülsenförmigen Elementes 52 verlaufende Nut 54 auf, deren Form deutlich aus Fig. 13 ersichtlich ist. Der Außendurchmesser des Teiles 52 ist dabei kleiner als der Innendurchmesser des zylindrischen Teiles 43 zu wählen.
Bei der Ausführungsform nach Fig. 10 ist demgegenüber
der Innendurchmesser des zylindrischen Teiles 39 etwas
größer als der Außendurchmesser des Teiles 43 gewählt,
da bei der Ausführungsform nach Fig. 10 das zylindrische
Teil 39 auf das Teil 43 aufgesetzt wird.

Bei der Ausführungsform einer Dosiereinrichtung nach Fig.12
und 13 wird nach dem Einsetzen des hülsenförmigen Teiles
52 die Schraube 51 durch das Gewinde 50 derart eingeschraubt,daß sie mit ihrem zur Kolbenstange 7 zugewandten
Ende zumindest teilweise in die Nut 54 eingreift. Damit
wird bei einer Drehung des hülsenförmigen Teiles 52 um
die Achse der Kolbenstange 7 herum infolge des Eingriffs
zwischen der Führungsnut 54 und der Führungsschraube 51
ein gleichzeitiges Verschieben des hülsenförmigen Teiles
52 in Richtung der Achse der Kolbenstange 7 aufgrund des
kurvenförmigen Verlaufes der Führungsnut 54 erreicht.
Fig. 13 zeigt, daß die Verstellung des hülsenförmigen
Teiles 52 derart vorgenommen werden kann, daß die Führungsschraube 51 die in Fig. 13 gestrichelt eingetragenen und
durch die Bezugszeichen 51a, 51b und 51c gestrichelt eingetragenen Hauptstellungen einnimmt. Die Stellung 51a entspricht derjenigen Stellung des hülsenförmigen Teiles 52
gegenüber dem ringförmigen Teil 53, in der die Rändelung
auf dem Rand 43' des Ringes aufliegt. In der Stellung 51b
ist das hülsenförmige Teil 52 um eine vorbestimmte Distanz
gegenüber dem Ring 43 angehoben und in der Stellung 51c
ist das hülsenförmige Teil 52 um eine noch größere Distanz
gegenüber dem Ring 43 angehoben als in der Stellung 51b.
Als Vergleich zu der Ausführungsform nach Fig. 10 sei
ausgeführt, daß die Stellung 51a der Führungsschraube 51
dem Zustand entspricht, in welchem bei der Ausführungsform nach Fig. 10 der Stift 41 in die Führungsnut 42a
eingreift, während die Stellung der Führungsschraube 51
gemäß Bezugszeichen 51b dem Eingriff des Stiftes 41 in
die Führungsnut 42b und die Position 51c der Stellung des Führungsstiftes 41 in der Führungsnut 42c entspricht.
Auf diese Weise wird entsprechend der Ausführungsform
nach Fig. 10 das zylindrische Teil 39 bzw. 52 in vorzugsweise drei möglichen Positionen in Richtung der Achse der
Kolbenstange 7 gegenüber der Halterung 30 arretierbar und
damit die Bewegung der Kolbenstange 7 mit dem Knopf 44
in drei unterschiedlichen Stellungen kurz vor Beendigung
des Ausspritzvorganges in Richtung des Pfeiles 36 in
Fig. 7 arretierbar. Wenn demzufolge bei Einstellung des
zylindrischen Teiles 39 bzw. 52 in einer der Positionen
42a,42b,42c bzw. 51a,51b,51c eine Arretierung der Kolbenstange 7 erhalten wird, die in der vorgenannten Reihenfolge
den Darstellungen nach Fig. 14a, Fig. 14b und Fig. 14c entspricht, d.h. daß in der ersten Position der Kolben 55
dicht oder nahezu dicht am Ausgang 56 des Spritzenkolbens
zu liegen kommt, während in der zweiten Position eine
größere Distanz zwischen dem Kolben 55 und dem Ausgang 56
eingehalten wird und in der dritten Position ein noch
größerer Abstand zwischen dem Kolben 55 und dem Spritzenzylinderausgang 56. Bei Anwendung der in Fig. 10 und Fig.12,
Fig. 13 dargestellten Ausführungsformen einer Dosiereinrichtung in Bezug auf einen Spritzenzylinder gleichen Volumens entsprechend Fig. 14a bis 14c lassen sich damit drei
Voluminas einstellen, wie dies in Fig. 14a bis 14c durch
$V_1$ bis $V_3$ angedeutet ist, während andererseits diese Voluminas $V_1,V_2$ und $V_3$, bezogen auf drei unterschiedliche Spritzenzylinder, d.h. auf drei Spritzenzylinder unterschiedlichen Durchmessers wiederum gleich groß sein können. Die
Wahl der Einstellmöglichkeiten der Dosiereinrichtung kann
beispielsweise derart getroffen sein, daß die erste Position
51a entsprechend Fig. 14a in Bezug auf den möglichen Einsatz von drei Spritzenzylinder unterschiedlicher Größe
entsprechend Fig. 2 zur Dosierung eines Volumens V bei
Einsatz des Spritzenzylinders größten Durchmessers dient,
während bei Fig. 14b entsprechend Position 51b das gleiche
Volumen für den Spritzenzylinder mittleren Durchmessers
erhalten wird, während entsprechend Fig. 14c und der

0100369

Position 51c das gleiche Volumen für den Spritzenzylinder kleinsten Durchmessers erhalten ist.

Durch Vornahme entsprechender Markierungen lassen sich die drei möglichen Einstellpositionen des zylindrischen Teiles 39 bzw. des Teiles 52 gegenüber der Halterung ohne weiteres erkennen, so daß die Handhabung und Einstellung von Dosierungsvolumen für das Reagens ohne weiteres möglich ist.

Die Halterung und/oder die Dosiereinrichtung können aus Metall und/oder Kunststoff hergestellt werden.

Im folgenden sei der Vorgang beim Aufziehen und Ausspritzen eines Spritzenzylinders mit der erfindungsgemäßen Halterung und Dosiereinrichtung erläutert. Nach dem Einsetzen eines Spritzenzylinders in die Halterung wird mittels eines Kupplungsteiles 7' (Fig.1a) an der Kolbenstange 7 eine Verbindung zwischen dem Kolben im Spritzenzylinder 2 und der Kolbenstange 7, z.B. durch einfaches Einschieben der Kolbenstange 7 in eine zugehörige komplementäre, nicht weiter dargestellte Aussparung im Kolben, hergestellt. Dann kann das Aufziehen der Spritze auf folgende Art und Weise vorgenommen werden:

Vor der Betätigung der Kolbenstange in Richtung des Pfeiles 36 in Fig.7 wird die Dosiereinstellung vorgenommen, d.h. eine der Positionen 42a bis 42c bzw. 51a bis 51c gewählt. Die Kolbenstange wird dann in Richtung des Pfeiles 36 (Fig.7) bis zu dem durch den Knopf 44 und die Wandung 40 des Teiles 39 bzw. 52 erreichbaren Anschlages betätigt, wobei der Kolben 55 bis in eine der in Fig. 14a bis Fig. 14c gezeigten Positionen abhängig von der vorgenommenen Dosiereinstellung verlagert wird. Dann wird die Spritze mit dem Reagens aufgezogen, wobei die aufgezogene Reagenzmenge größer gewählt ist als die gewünschte Dosierung, anschließend wird die Spritze umgedreht, d.h. mit der Öffnung 56 nach oben und die im Spritzenzylinder befindliche Luft und teilweise im Spritzenzylinder befindliches überschüssiges Reagens ausgespritzt, indem die Kolbenstange 7 wieder bis in eine der Stellungen nach Fig. 14a bis 14c in Richtung des Pfeiles 36 bewegt wird, wobei diese Position ersichtlicherweise von der vorgenommenen Einstellung der Dosiereinrichtung abhängt. Anschließend wird dann Blut in den Spritzenzylinder aufgezogen, indem die Kolbenstange 7 entgegen der Richtung des Pfeiles 36 herausgezogen wird, und zwar so weit, bis das erforderliche Volumen durch Ablesung auf der Eichskala des Spritzenzylinders aufgezogen wurde. Anschließend wird das gesamte Volumen im Spritzenzylinder nach vorheriger Zurückstellung der Dosiereinrichtung in die Null-Position aus dem Spritzenzylinder ausgespritzt, so daß kein Restvolumen im Spritzenzylinder verbleibt. Die Nullstellung erfolgt dabei beispielsweise derart, daß das Teil 39 bzw. 52 so verstellt wird, daß der Stift 41 in die Führungsnut 42a bzw. die Führungsschraube 51 in die Position 51a verbracht wird.

Eine weitere Methode zum Aufziehen des Reagens und des Blutes ist wie folgt: Die Dosiereinstellung entspricht der Nullstellung und die Kolbenstange 7 wird vollständig in den Spritzenzylinder in Richtung des Pfeiles 36 verschoben. Dann wird das Reagens aufgezogen, indem die Kolbenstange entgegen dem Pfeil 36 aus dem Spritzenzylinder herausgezogen wird, und zwar über eine Länge, die ein größeres Volumen bzw. eine größere Menge an Reagens sicherstellt, als dosiert werden soll. Anschließend wird die Dosiereinrichtung durch entsprechende Verstellung des Teiles 39 bzw. 52 auf die gewünschte Dosiermenge eingestellt und die Kolbenstange 7 bei umgedrehtem Spritzenzylinder, d.h. mit der Öffnung 56 bzw. der nicht gezeigten Nadel nach oben wiederum in Richtung des Pfeiles 36 bis zu dem durch die Einstellung der Dosiereinheit vorgegebenen Anschlagstellung bewegt. Sobald bei vorgenommener Dosiermengeneinstellung die Endposition der Kolbenstange 7 bei ihrer Bewegung in Richtung des Pfeiles 36 infolge des Anschlages zwischen dem zylindrischen Bodenteil 40 und dem Knopf 44 erhalten ist, also die gewünschte Dosiermenge im Spritzenzylinder vorhanden ist, wird die Kolbenstange 7 wieder in entgegengesetzter Richtung zum Pfeil 36 unter gleichzeitigem Aufziehen von Blut in den Spritzenzylinder bewegt, bis das gewünschte Gesamtvolumen aufgezogen ist. Dann wird die Dosiereinrichtung wieder in Nullstellung verbracht und der gesamte Inhalt des Spritzenzylinders ausgespritzt. Nach dem Ausspritzen wird die Verbindung zwischen dem Kolben und dem Kupplungsteil 7' der Kolbenstange 7, z.B. durch ruckartiges Zurückziehen der Kolbenstange 7, gelöst, so daß dann der benutzte Spritzenzylinder 2 aus der Halterung entnommen werden kann.

Es ist zu beachten, daß an sich nur das Aufziehen einer relativ kleinen Reagenzmenge mit Ableseschwierigkeiten verbunden ist und in Ungenauigkeiten resultieren kann, weil die aufzuziehende Reagenzmenge etwa einem Fünftel des aufzuziehenden Blutvolumens entspricht. Das nachfolgende Aufziehen von Blut in den Spritzenzylinder ist dagegen weniger mit Ungenauigkeiten verbunden, bedingt durch die größere Blutmenge und die leichtere Ablesung des aufgezogenen Blutvolumens.

Die Erfindung schafft eine Vorrichtung, genauer genommen eine Halterung für den Einsatz von Spritzenzylindern unterschiedlicher Größe zusammen mit einer Dosiereinrichtung. Die Dosiereinrichtung läßt sich auf verschiedene Weise ausbilden, so daß die unter Bezugnahme auf Fig.10, 11 und Fig. 12,13 beschriebenen Einheiten nur als Ausführungsbeispiele zu betrachten sind. Grundsätzlich ist ein Teil mit einem nockenförmigen Glied und ein Teil mit einer Führungskurve vorzusehen, die gegeneinander in Richtung der Achse der Kolbenstange 7 und vorzugsweise gleichzeitig um die Achse der Kolbenstange 7 relativ zueinander drehfähig sind. Durch die Verstellung dieser
beiden Teile läßt sich eine Dosiereinstellung vornehmen,
wobei die Dosiereinstellung selbst beispielsweise dadurch
erreicht wird, daß die Kolbenstange, ein an der Kolbenstange angeordneter Anschlag oder der Knopf 44 der Kolbenstange entweder mit dem Teil, das den Nocken trägt, oder
mit demjenigen Teil, in dem die Führungskurve ausgebildet
ist, zusammenwirkt.

Aus vorstehender Beschreibung ist ferner ersichtlich, daß
die Einrichtung zur Dosierungseinstellung auf der
Halterung 30, vorzugsweise in Richtung auf das den Knopf 44
tragende Ende der Kolbenstange 7 verstellbar angeordnet ist und
gemäß den beschriebenen Ausführungsformen im wesentlichen konzentrisch und außenliegend zur Kolbenstange 7 vorgesehen
ist.

Die erfindungsgemäße Vorrichtung läßt sich auch als Blutabnahmegerät einsetzen und der Spritzenzylinder als Behältnis zum Transport des abgenommenen Blutes verwenden. Wenn
in den Spritzenzylinder Blut gemäß der vorstehenden Beschreibung aufgezogen wurde, wobei mittels der Dosiereinrichtung vor dem Aufziehen von Blut ein Reagens in
den Spritzenzylinder mit einem exakten Volumen aufgezogen
worden ist, braucht nach dem Aufziehen von Blut das
Gemisch aus Blut und Reagens nicht aus dem Spritzenzylinder
in ein Behältnis ausgespritzt zu werden. Der Spritzenzylinder 2 wird nach Lösung der Kolbenstange 7 vom Kolben von
der Halterung entfernt; ferner wird nach Abziehen der
Nadel von der Öffnung 56 diese Öffnung mit einem nicht
dargestellten Verschlußmittel verschlossen.Dann wird ein
derart verschlossener und eine Mischung aus Blut und
Reagens enthaltender Spritzenzylinder direkt zum Labor
transportiert und dort in eine Zentrifuge eingesetzt, ohne
daß zusätzliche Behälter oder Glasröhrchen etc. erforderlich werden bzw. ein Umfüllen aus dem Spritzenzylinder in
derartige Behältnisse notwendig wird. Im Bedarfsfall kann die rückwärtige große Öffnung 26 des Spritzenzylinders
noch durch eine zusätzliche Kappe abgeschlossen werden.
Es ist ersichtlich, daß mit der erfindungsgemäßen Vorrichtung der optimale Einsatz von Spritzenzylindern unterschiedlicher Größe möglich ist.

Um zu verhindern, daß der Kolben aus dem Spritzenzylinder 2
heraus verlagerbar ist, wird der Spritzenzylinder 2 vorzugsweise nahe seiner Öffnung 26 mit einer nach innen
verlaufenden ringförmigen Wulst versehen, die als Begrenzung für die Bewegung des Kolbens in Richtung auf die
Öffnung 26 dient. Ersichtlicherweise können auch andere
Mittel vorgesehen sein, die eine Verlagerung des Kolbens
aus dem Spritzenzylinder 2 durch die Öffnung 26 heraus
verhindern. Die Lösung zwischen Kolbenstange 7 und Kolben
erfolgt vorteilhafterweise durch einen kurzen Ruck, der
auf die Kolbenstange 7 entgegengesetzt zur Richtung des
Pfeiles 36 in Fig.7 ausgeführt wird. Der Kolben, der in
Fig. 14 mit 55 bezeichnet ist, bleibt nach Entfernung des
Spritzenzylinders 2 aus der Halterung nahe der Öffnung 26,
wie vorstehend beschrieben.

# REINHARD , KREUTZ & SKUHRA  0100369

## PATENTANWÄLTE

Reinhard, Kreutz & Skuhra · Leopoldstraße 51 · D-8000 Munchen 40

DR. ERNST STURM (1951-1980)
DR. HORST REINHARD
DIPL.-ING. KARL JÜRGEN KREUTZ
DIPL.-ING. UDO SKUHRA

LEOPOLDSTRASSE 51
D-8000 MÜNCHEN 40

TELEFON     : 0 89 / 33 40 78
TELEX       : 5 21 28 39 isar d
TELEGRAMM : ISARPATENT

| Unser Zeichen/our ref | Ihr Zeichen/your ref. | Datum/date |
|---|---|---|
| P1633 S/br | | 29.Juli 1982 |

Dr. Nikola Suwandschieff, Bludenz/Österreich

## Patentansprüche

1. Vorrichtung, insbesondere zur Blutabnahme, bestehend aus einer Halterung und einem an der Halterung befestigbaren Spritzenzylinder, wobei die Halterung eine Führung für die Kolbenstange aufweist, d a d u r c h   g e k e n n z e i c h n e t , daß die Halterung (1; 30) eine Einheit (3,4,5; 11,12; 15,16,22,23; 31,32,33) zur Befestigung von Spritzenzylindern (2) unterschiedlichen Durchmessers und/oder Volumens aufweist, und daß eine Einrichtung (38,39,43, 52) zur Begrenzung der Bewegung der Kolbenstange (7) in Ausspritzrichtung zur Einstellung einer Dosierungsmenge $(V_1, V_2, V_3)$ vorgesehen ist.

2. Vorrichtung nach Anspruch 1, dadurch gekennzeichnet, daß die Einheit (3,4,5; 11,12; 20 bis 23; 31,32,33) ein Adapterelement zur Aufnahme von Spritzenzylindern (2) unterschiedlichen Durchmessers ist.

3. Vorrichtung nach einem der vorangehenden Ansprüche,
dadurch gekennzeichnet, daß das Adapterelement mehrere
konzentrische, ringförmig verlaufende Vorsprünge (3,4)
aufweist.

4. Vorrichtung nach Anspruch 3, dadurch gekennzeichnet,
daß die Vorsprünge (3,4) mit jeweils wenigstens einem
Paar von Rastöffnungen (5) versehen sind und die
Spritzenzylinder (2) in die Rastöffnungen (5) einrastbare, seitlich abstehende Rastnocken (6) tragen.

5. Vorrichtung nach Anspruch 1 bis 4, dadurch gekennzeichnet, daß das Adapterelement einen einzigen Ring (3)
aufweist, und daß die Spritzenzylinder (2) unterschiedlichen Durchmessers und/oder Volumens an ihrem offenen
Ende (2a) einen Flanschabschnitt (11) mit seitlich abstehenden Rastnocken tragen, und daß jeder Flanschabschnitt (11) einen vorbestimmten, dem Innendurchmesser
des Ringes (3) entsprechenden Außendurchmesser aufweist.

6. Vorrichtung nach wenigstens einem der vorangehenden Ansprüche, dadurch gekennzeichnet, daß die Einheit zur
Befestigung von Spritzenzylindern wenigstens ein Paar
etwa parallel zur Achse des Spritzenzylinders (2) verlaufende, von der Halterung (1) in Richtung auf den
Spritzenzylinder (2) gewandte, abstehende Rastnocken
(15,16) aufweist, welche in entsprechende, in jeweils
einem Flansch (20,21) des Spritzenzylinders ausgebildete
Öffnungen (22,23) einrastbar sind.

7. Vorrichtung nach wenigstens einem der vorangehenden
Ansprüche, dadurch gekennzeichnet, daß die Halterung
(30) mehrere, senkrecht zur Achse der Kolbenstange verlaufende Schlitze (31,32,33) zur Aufnahme von Spritzenzylindern (2) unterschiedlichen Durchmessers mit deren

0100369

Flanschabschnitten (20,21) aufweist.

8. Vorrichtung nach wenigstens einem der vorangehenden Ansprüche, dadurch gekennzeichnet, daß die Einrichtung (38,39,43,52) zur Begrenzung der Kolbenstangenbewegung ein Teil (43,52) mit einer Führungskurve und ein Teil (39,43) mit einem Führungsnocken (41,51) aufweist.

9. Vorrichtung nach Anspruch 8, dadurch gekennzeichnet, daß eines der beiden Teile mit Führungskurve bzw. Führungsnocken gegenüber dem anderen Teil mit Führungsnocken bzw. Führungskurve verstellbar ist.

10. Vorrichtung nach Anspruch 8 oder 9, dadurch gekennzeichnet, daß die Führungskurve mehrere Raststellungen (42a,42b,42c; 51a,51b,51c) aufweist.

11. Vorrichtung nach Anspruch 8 bis 10, dadurch gekennzeichnet, daß die Einrichtung (38,39,43,52) an der Halterung (30) und im wesentlichen konzentrisch zur Kolbenstange (7) ausgebildet ist.

12. Vorrichtung nach wenigstens einem der vorangehenden Ansprüche, dadurch gekennzeichnet, daß die Kolbenstange (7) gegenüber dem Kolben (55) lösbar ist.

13. Vorrichtung nach Anspruch 12, dadurch gekennzeichnet, daß die Kolbenstange (7) zum Lösen gegenüber dem bzw. zur Verbindung mit dem Kolben (55) ein Kupplungsteil (7') trägt.

Fig.1a

Fig.1b

Fig.1c

0100369

2/6

Fig.3a

Fig.3b

Fig.3c

Fig.3d

Fig. 2

Fig. 4a

Fig. 4b

**Fig.5**

**Fig.6**

*36*

*VIII*  *VIII'*

*31*  *30*

*32*  *39*

*33*  *38*

*40*

*7*

*Fig.7*

*1*
*2*
*3*
*4*
*5*
*ml*

*20*  *21*

*Fig.9*

*35*

*B*

*Fig.8*

0100369

Fig.10

Fig.11

Fig.12

Fig.13

Fig.14c

Fig.14b

Fig.14a

## Europäisches Patentamt

## EUROPÄISCHER RECHERCHENBERICHT

| | EINSCHLÄGIGE DOKUMENTE | | EP 82106910.1 |
|---|---|---|---|
| **Kategorie** | **Kennzeichnung des Dokuments mit Angabe, soweit erforderlich, der maßgeblichen Teile** | **Betrifft Anspruch** | **KLASSIFIKATION DER ANMELDUNG (Int. Cl. ³)** |
| X,Y | <u>DE - A1 - 3 028 432</u> (G. REITZ)<br>* Gesamt *<br><br>-- | 1-6,8,<br>9,11 | A 61 M 5/31<br>A 61 M 5/315<br>A 61 M 5/24 |
| Y | <u>DD - A - 57 928</u> (O. KAISER)<br>* Gesamt *<br><br>-- | 1-6,8,<br>9,11 | A 61 B 5/14 |
| E,X | <u>DE - U1 - 81 05 181.6</u> (G. REITZ)<br>* Gesamt *<br><br>-- | 1-11 | |
| E,X | <u>DE - A1 - 3 106 988</u> (G. REITZ)<br>* Gesamt *<br><br>-- | 1-7 | |
| A | <u>US - A - 3 815 785</u> (R. GILMONT)<br>* Gesamt *<br><br>-- | 1,8-11 | **RECHERCHIERTE SACHGEBIETE (Int. Cl. ³)** |
| A | <u>DE - A1 - 2 717 830</u> (BECTON-<br>DICKINSON)<br>* Seite 9, 2. Absatz; Seite<br>16, 2. Absatz - Seite 18,<br>1. Absatz *<br><br>-- | 12,13 | A 61 B 5/00<br>A 61 M 5/00 |
| A | <u>DE - A1 - 2 753 609</u> (J. SILVER)<br>* Patentanspruch 1 *<br><br>---- | | |

Der vorliegende Recherchenbericht wurde für alle Patentansprüche erstellt.

| Recherchenort | Abschlußdatum der Recherche | Prüfer |
|---|---|---|
| WIEN | 21-04-1983 | LUDWIG |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82